# FASCICULE DE BREVET EUROPEEN

(11) **EP 0 635 036 B1**
(45) Date de publication et mention de la délivrance du brevet: **14.05.1997**
(21) Numéro de dépôt: 93909017.1
(22) Date de dépôt: 09.04.1993
(51) Int. Cl.: C08G 65/26

(54) **PROCEDE DE PREPARATION D'AMINES POLYOXYALKYLEES**
VERFAHREN ZUR HERSTELLUNG VON POLYOXYALKYLENAMINEN
PROCESS FOR THE PREPARATION OF POLYOXYALKYLATED AMINES

(30) Priorité: 10.04.1992 FR 9204673
(43) Date de publication de la demande: 25.01.1995
(73) Titulaire: CHRYSO S.A., F-91383 Chilly-Mazarin (FR)
(72) Inventeur: MOSQUET, Martin, F-45300 Pithiviers (FR); GUICQUERO, Jean-Pierre, F-94440 Santeny (FR); LE PERCHEC, Pierre, F-69003 Lyon (FR); CHEVALIER, Yves, F-69540 Irigny (FR)
(74) Mandataire: Tilloy, Anne-Marie
(86) Numéro de dépôt international: FR9300364
(87) Numéro de publication internationale: WO9321254

(56) Documents cités:
- EP-A- 0 149 795
- EP-A- 0 268 920
- EP-A- 0 373 860
- CHEMICAL ABSTRACTS, vol. 87, no. 18, 31 Octobre 1977, Columbus, Ohio, US; abstract no. 136821x, AKIMOTO ET AL 'Pentaerythritol Polyether' page 33
- POLYMER SCI. USSR vol. 22, no. 11, 1980, OXFORD, GB pages 2279-2786 PTITSYNA N.V. et AL 'Synthesis of Polyethylene Glycols of Molecular Weight Above 10,000 By Anionic Polymerisation'

## Description

La présente invention concerne un procédé de préparation de composés comportant au moins une fonction amine primaire ou secondaire et au moins une chaîne polyoxyalkylée.

Pour la synthèse de tels composés, plusieurs voies ont été proposées dans l'art antérieur.

La première de ces voies consiste à traiter une molécule polyoxyalkylée comportant un groupement terminal apte à être transformé en une fonction amine.

Ainsi, il est connu de préparer des amines polyoxyéthylées à partir de polyéthylène glycol. L'ouvrage "Eur. Polym. J. Vol. 19, n°12, p. 1177 - 1183, 1983" de Zalipsky décrit la préparation d'amino-polyéthylène glycol à partir de polyéthylène glycol en modifiant la fonction hydroxyle terminale en un atome de chlore, puis en remplaçant cet atome de chlore par une fonction azidure que l'on réduit en présence d'un catalyseur.

D'autres procédés plus récents comme celui de Yinglin, dans "Synthetic Communications 21,(1), 79-84 (1991)" effectuent la préparation d'amino-polyéthylène glycol à partir de polyéthylène glycol. Selon ces procédés, on fixe sur la fonction hydroxyle terminale un radical tosyle puis on fait réagir le produit obtenu avec du phtalimidure de potassium ou du diformamidure de sodium pour aboutir au composé aminé recherché.

Le brevet belge n°849343 décrit la préparation de polyoxyéthylènes d'amines secondaires répondant à la formule (I) suivante :

HN - [(CHX-CHY-O)ₙ - R]₂

dans laquelle :
- R est un radical alkyle, saturé ou non, ayant de 9 à 24 atomes de carbone, un radical cyclohexyle ou un radical aryle éventuellement porteur de groupes alkyles,
- X et Y représentent chacun un atome d'hydrogène ou un radical méthyle mais ne peuvent désigner en même temps un radical méthyle et,
- n est un nombre entier de 1 à 15 ou, lorsque R est un radical phényle ou tosyle, un nombre entier de 2 à 15.

Pour préparer ces composés, on fait réagir des oxalkylates répondant à la formule (II) suivante :

R - (O-CHX-CHY)ₙ - OH

dans laquelle R, X, Y et n ont les significations données ci-dessus, en phase liquide, avec de l'ammoniac et de l'hydrogène en présence de catalyseurs d'hydrogénation-déshydrogénation, à une température de 150°C à 250°C et sous une pression absolue de 0,5 à 1,5 atmosphère.

De tels procédés, lorsqu'ils sont économiquement acceptables, présentent toutefois l'inconvénient principal de nécessiter plusieurs étapes car l'amination ne peut être réalisée qu'en dernier lieu, lorsque la partie polyoxyéthylée a atteint la longueur désirée.

Une deuxième voie de synthèse d'amines polyoxyalkylées consiste alors à préparer les composés répondant à la formule (I) en additionnant de l'oxyde de propylène ou d'éthylène sur des molécules comportant déjà une fonction amine.

Ceci conduit généralement à des additions d'oxyde sur l'atome d'azote de la fonction amine qui perd alors son caractère primaire ou secondaire.

Pour éviter la polyoxyalkylation sur l'atome d'azote de la fonction amine, il a été proposé de partir d'amines secondaires ou primaires protégées par encombrement stérique à l'aide d'un groupement ramifié.

Ainsi, le brevet américain n°2 871 266 décrit un procédé de préparation d'amines secondaires mono(polyoxyalkylées) à partir d'amines encombrées sous la forme de tert-carbinamine de formule : 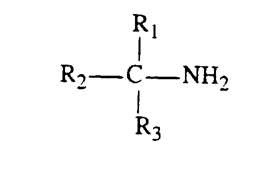 dans laquelle R1, R2 et R3 sont des radicaux alkyles dont le nombre total d'atomes de carbone varie de 7 à 23.

Pour former l'amine secondaire mono(polyoxyalkylée), il est nécessaire de former au préalable un dérivé N-hydroxyéthylé à partir de cette tert-carbinamine puis de faire réagir ce produit intermédiaire avec de l'oxyde d'éthylène ou de propylène en présence d'un catalyseur alcalin.

Un tel procédé présente donc l'inconvénient de nécessiter la présence d'un carbone encombré en alpha de l'azote par des groupements comportant au moins 7 atomes de carbone et ne permet d'obtenir que des amines secondaires polyoxyalkylées dont l'hydrogène est peu réactif.

Une troisième voie de synthèse des amines polyoxyalkylées consiste alors à partir d'un amino-alcool dont on a protégé la fonction amine en la transformant en une fonction inerte avec les époxydes, puis à effectuer l'addition d'oxyde de propylène ou d'éthylène.

Cette troisième voie est notamment décrite dans "Makromol. Chem. 184, 1849-1859, 1983" dans le cas de la préparation de polyoxiranes comportant des fonctions amines primaires terminales à partir d'amino-alcool.

La fonction amine de l'amino-alcool de départ est au préalable protégée au moyen d'un groupement succinyle. On forme ensuite un alcoolate de potassium à l'aide de dihydronaphtylidure de potassium. L'alcoolate peut alors ouvrir l'oxyde d'éthylène et l'on obtient l'amine primaire polyoxyéthylée recherchée après une polyaddition d'oxyde d'éthylène suivie d'une neutralisation, d'une régénération de la fonction amine puis d'une élimination des produits secondaires issus de la régénération.

Il n'existe donc pas dans l'art antérieur de procédé permettant de préparer des composés comportant au moins une fonction amine primaire ou secondaire et au moins une chaîne polyoxyalkylée qui ne comporte pas d'étape de protection des fonctions amines.

La demanderesse, en collaboration avec le CNRS et le GIE LAFARGE COPPEE RECHERCHE, a mis au point un procédé simple de préparation de composés pourvus d'au moins une fonction amine primaire ou secondaire et d'au moins une chaîne polyoxyalkylée. De façon surprenante, ce procédé ne comporte aucune étape de protection des fonctions amines.

Le procédé de la présente invention se caractérise en ce que l'on fait réagir :
- au moins un amino-alcoolate (B) obtenu à partir d'un amino-alcool (A) comportant au moins un groupement OH et au moins une fonction amine primaire ou secondaire, par remplacement d'une fraction ou de la totalité des groupements OH par des groupements OM, M étant choisi parmi le sodium, le potassium, le rubidium et le césium, lesdits amino-alcool (A) et amino-alcoolate (B) étant dépourvus de groupements susceptibles de réagir entre eux ou avec une fonction portée par l'amino-alcool (A) ou l'amino-alcoolate (B) dans les conditions réactionnelles du procédé, tels qu'un groupement acide ou un halogène ;
- avec un oxirane, un mélange d'oxiranes ou une séquence d'oxiranes, dans un solvant aprotique et anhydre, sous atmosphère anhydre et à température comprise entre 0°C et 200°C, de façon à fixer sur chaque atome d'oxygène provenant, soit d'un groupement OH, soit d'un groupement OM , une série de "m" motifs semblables ou différents, m étant un nombre entier supérieur ou égal à 1, de préférence compris entre 1 et 10 000, en particulier compris entre 1 et 500 et spécialement compris entre 1 et 250, lesdits "m" motifs répondant à la formule suivante :

   -[C (R₁)(R₂) - C(R₃)(R₄)-O]-
- dans laquelle R₁, R₂, R₃ et R₄ sont semblables ou différents et peuvent être choisis dans le groupe constitué par :
   * l'atome d'hydrogène,
   * les radicaux monovalents, saturés ou non, alkyle, cyclo-alkyle ou aryle,
   * lesdits radicaux renfermant éventuellement divers substituants non réactifs vis-à-vis d'une fonction amine et d'une fonction alcoolate, tels que des fonctions éthers ou des amines tertiaires.

Le procédé selon l'invention est éventuellement suivi d'une deuxième étape au cours de laquelle on peut neutraliser ou fonctionnaliser le produit obtenu précédemment de façon connue en soi, en fixant sur chaque atome d'oxygène terminant une chaîne polyoxyalkylée, par exemple un atome d'hydrogène, un radical éventuellement substitué, saturé ou non, alkyle ou cycloalkyle, ou un radical aryle.

Le procédé selon l'invention peut également être poursuivi par d'autres additions d'un ou de plusieurs oxiranes de nature chimique différente, de préférence dans les conditions préconisées dans l'invention. De la sorte, on prépare des chaînes polyoxyalkylées comportant des séquences d'oxiranes différents d'une séquence à une autre.

Par fonction alcoolate de potassium, de rubidium, de césium ou de sodium, on entend dans la présente description, respectivement les groupements OK, ORb, OCs ou ONa portés par un atome de carbone.

Le procédé selon l'invention présente l'avantage d'être sélectif tout en étant simple, de pouvoir être mis en oeuvre dans des conditions opératoires douces, avec des produits de départ couramment employés en synthèse organique, et de bien se prêter à une application industrielle tant sur le plan de la rentabilité que de la sécurité.

En outre, il permet le contrôle du degré de polyoxyalkylation, ce qui offre l'avantage de pouvoir faire varier le caractère hydrophile ou hydrophobe de la molécule. En effet, du fait que l'introduction d'oxirane peut être réalisée progressivement dans le milieu réactionnel, il suffit de l'interrompre dès que la longueur moyenne des chaines polyoxyalkylées souhaitée est atteinte.

Le procédé selon l'invention est basé sur la découverte surprenante qu'il est possible d'obtenir de façon hautement sélective l'ouverture des oxiranes par les seules fonctions alcoolates, sans protection préalable des fonctions amines primaires ou secondaires du produit de départ, ni recours à un catalyseur.

Ce résultat est obtenu conformément à l'invention lorsque les fonctions alcoolates sont des fonctions alcoolates de sodium, de potassium, de rubidium ou de césium. On a en effet trouvé que la polyaddition s'effectue sélectivement sur les atomes d'oxygène provenant d'un groupement OH ou d'un groupement OM, à la condition que le contre-ion M soit un cation sodium, potassium, rubidium ou césium. La plupart des fonctions amines primaires et/ou secondaires, quoique non protégées, peuvent donc être conservées.

En revanche, si on effectue la réaction à partir d'un amino-alcool (A), autrement dit lorsque le produit de départ ne comporte pas de groupement OM, on perd la sélectivité de la réaction et en conséquence la polyaddition peut se produire sur la ou les fonctions amines primaires ou secondaires du produit de départ.

Au travers des essais suivants donnés à titre illustratif et non limitatif, nous allons présenter les avantages de l'invention.

A partir d'un amino-alcool (A), que l'on soumet à l'action d'une base, on prépare un amino-alcoolate (B) (par transformation des groupements OH en groupements OM), on réalise une polyoxyalkylation avec de l'oxyde d'éthylène dans le tétrahydrofuranne.

Les amino-alcoolates ont été préparés à l'aide de différentes bases dans le but de comparer l'influence de la nature du cation M associé à l'amino-alcoolate.

Les conditions opératoires, autres que celles présentées dans le tableau suivant, sont les mêmes pour ces quatre essais :

| N° | Amino-alcoolate (B) | C1 | C2 | B⁻ | F (%) |
|---|---|---|---|---|---|
| 1 | ⁻OCH₂CH₂NH₂ | 215,17 | 2,15 | DPMK⁺ | <3 % |
| 2 | ⁻OCH₂CH₂NHCH₃ | 215,0 | 2,15 | DPMK ⁺ | <3 % |
| 3 | ⁻OCH₂CH₂-NHCH₃ | 210,0 | 2,10 | DPMNa⁺ | 45^{(a)} |
| 4 | ⁻OCH₂CH₂-NHCH₃ | 207,0 | 2,07 | NaNH₂ | 55^{(a)} |
| Notations : - C1 : concentration en amino-alcoolate (B) dans le milieu (exprimée en 10⁻³ mole par litre), - C2 : concentration en oxyde d'éthylène dans le milieu (exprimée en mole par litre), - B⁻ : base utilisée pour générer la fonction alcoolate à partir de la fonction alcool, - DPMK⁺ : diphénylméthylpotassium - DPMNa⁺ : diphénylméthylsodium - F : fraction molaire d'amino-alcoolate (B) dont la fonction amine a perdu son caractère primaire ou secondaire de départ, - (a) les polyadditions sont dans ces cas plutôt lentes. Les valeurs de F sont alors mesurées pour des consommations partielles d'oxirane de 45 % et de 50 %, respectivement pour les polyadditions des essais 3 et 4. Ces valeurs de F, élevées, résultent de l'addition d'oxirane sur l'azote de la fonction amine. | | | | | |

Il apparaît une grande différence de sélectivité selon que le cation intervenant est le cation sodium ou le cation potassium. On observe en effet une sélectivité totale lorsque la fonction alcoolate réagissant avec l'oxirane, en l'occurrence l'oxyde d'éthylène, est une fonction alcoolate de potassium.

D'une manière générale, il est préférable, pour mettre en oeuvre le procédé selon l'invention, de partir d'un amino-alcoolate (B) dont les cations associés aux fonctions alcoolates sont des cations potassium, rubidium ou césium. Il est plus particulièrement avantageux de partir d'un amino-alcoolate (B) dont les cations sont des cations potassium.

Les amino-alcools (A) pouvant servir de produits de départ pour la préparation de l'amino-alcoolate (B) répondent avantageusement à la formule (III) suivante :

[(HNRi)ⱼQ](OH)_{(r +q)}

dans laquelle :
- j est le nombre de fonctions amines primaires ou secondaires qui sont identiques ou différentes les unes des autres, j variant de 1 à 10 inclus,
- r est la somme des nombres de groupements OH portés par l'ensemble des Ri et q est le nombre des groupements OH portés par Q, r+q étant supérieur ou égal à 1 et pouvant atteindre 10,
- Q représente un radical organique comportant de 2 à 50 atomes de carbone,
- chaque Ri est un atome d'hydrogène ou un radical organique comportant de 1 à 50 atomes de carbone,
- quand l'amino-alcool (A) renferme plus d'une fonction amine primaire ou secondaire, les Ri peuvent être semblables ou différents entre eux,
- Q et Ri peuvent être porteurs d'hétéroatomes et/ou comporter des substituants divers tels que des fonctions éthers ou amines tertiaires,
- Q, N et les Ri peuvent former ensemble un ou plusieurs cycles, ce ou ces cycles pouvant en outre contenir un ou plusieurs autres hétéroatomes,
- Q et les Ri doivent être dépourvus de groupements susceptibles de réagir entre eux ou avec d'autres groupements portés par l'amino-alcool (A) ou l'amino-alcoolate (B) dans les conditions réactionnelles du procédé selon l'invention.

Parmi les amino-alcools (A), on peut citer, à titre d'exemple, les composés couramment utilisés suivants :
- l'éthanol-amine
- la diéthanol-amine
- les propanol-amines
- la N-méthyléthanol-amine
- l'amino-4-cyclohexanol
- le diamino-propanol-2
- la N-décyléthanolamine.

L'amino-alcoolate (B) nécessaire pour la mise en oeuvre du procédé selon l'invention peut être préparé conformément à toute méthode connue de préparation d'un alcoolate de sodium, de potassium, de césium ou de rubidium. De préférence, on prépare l'amino-alcoolate (B) par mise en contact d'un amine-alcool (A) comportant au moins un groupement OH et au moins une fonction amine primaire ou secondaire avec une base apportant le ou les cations désirés.

La base utilisée est de préférence le diphénylméthylpotassium, l'hydrure de potassium, l'amidure de potassium ou le potassium finement divisé. En effet, de telles bases présentent l'avantage de transformer quantitativement les fonctions alcools en fonctions alcoolates à température modérée, par exemple à 30°C. L'emploi de telles bases permet en outre de préparer les amino-alcoolates (B) dans le solvant où sera conduit le procédé selon l'invention. Des bases telles que l'hydroxyde de sodium, de potassium, de rubidium ou de césium ou les alcoolates légers de sodium, de potassium, de rubidium ou de césium pourraient également être utilisées. Mais dans ce cas, la réaction de formation de la fonction alcoolate OM présente quelques inconvénients :
- cette réaction est équilibrée,
- cette réaction conduit à la formation d'eau ou d'alcool léger qu'il est nécessaire d'éliminer du mélange réactionnel, par exemple par évaporation, avant d'entreprendre la réaction de polyoxyalkylation car sinon une partie des oxiranes serait consommée par cette eau ou ces alcools légers.

Conformément au procédé selon l'invention, il n'est pas nécessaire que tous les groupements OH du produit de départ aient été transformés en groupements OM. La polyoxyalkylation s'effectue quand même sur tous les atomes d'oxygène, qu'ils appartiennent à un groupement OH ou à un groupement OM.

Il est toutefois recommandé, pour obtenir une sélectivité satisfaisante, que le nombre des groupements OM soit supérieur à 30 %, de préférence supérieur à 40 % de la somme du nombre des groupements OM et du nombre des groupements OH.

Ces pourcentages sont particulièrement avantageux et procurent une sélectivité élevée lorsque M est le cation potassium. Néanmoins, ils peuvent varier suivant les conditions opératoires et le type de cation M intervenant. . De même, l'adjonction au solvant de composés à propriétés complexantes tels que des cryptates, peut entraîner ces pourcentages vers le bas.

En d'autres termes, pour mettre en oeuvre le procédé selon l'invention et déboucher sur un produit final à chaînes polyoxyalkylées liées essentiellement à des atomes d'oxygène et non à des atomes d'azote provenant d'une fonction amine, il est recommandé de partir du produit de la réaction de l'amino-alcool (A) avec une quantité de base telle que le rapport du nombre de cations M apportés par la base sur le nombre de groupements OH de l'amino-alcool (A) soit supérieur à 0,3 et de préférence supérieur à 0,4. Lorsque ce rapport est inférieur à 1, le produit obtenu est un mélange d'amino-alcoolates (B) différant par le nombre de groupement OH remplacés par des groupements OM. Ce mélange peut même encore renfermer de l'amino-alcool (A). C'est le cas notamment lorsque l'amino-alcool (A) de départ ne comporte qu'un seul groupement OH.

Parmi les oxiranes convenant à l'invention, on peut citer l'oxyde d'éthylène, l'oxyde de propylène, l'oxyde de butylène, l'oxyde d'amylène, l'oxyde d'octylène, l'oxyde de styrène, l'oxyde de méthylstyrène, l'oxyde de cyclohexane, ainsi que leurs divers dérivés substitués. Il est également possible d'utiliser un mélange de ces oxiranes ou plusieurs oxiranes différents successivement, de manière à réaliser une alternance de chaînes polyoxyalkylées de nature chimique différente.

De préférence, l'oxirane mis en oeuvre est choisi dans le groupe constitué par l'oxyde d'éthylène, l'oxyde de propylène, l'oxyde de styrène et leurs mélanges. De préférence encore, on choisit l'oxyde d'éthylène.

La quantité d'oxirane à ajouter n'est fonction que de la longueur des chaînes désirée et du nombre de groupements OH ou OM. Elle est indépendante de la quantité de base ayant servi à préparer le produit de départ.

Conformément à l'invention, le procédé est conduit dans un solvant aprotique et anhydre, ceci afin de ne pas régénérer les fonctions alcools.

Par solvant aprotique, on entend ici un solvant dépourvu d'hydrogène acide dans les conditions opératoires.

Le solvant est choisi de préférence parmi les solvants à forte polarité afin de faciliter la dissociation des cations sodium, potassium, rubidium ou césium des fonctions alcoolates. Les solvants convenant particulièrement bien au procédé selon l'invention sont le tétrahydrofuranne, le diglyme (diéthylène glycol diméthyl éther), le diméthylsulfoxyde ou l'hexaméthylphosphoreamide.

La réaction d'addition de l'oxirane sur les atomes d'oxygène est conduite de façon connue en soi, comme toute réaction d'addition d'un oxirane sur un alcoolate.

La réaction de polyaddition doit être effectuée sous atmosphère anhydre afin d'éviter l'introduction d'eau dans le milieu réactionnel qui régénèrerait les fonctions alcools et perturberait donc la polyaddition. On pourra pour cela travailler sous azote, sous argon ou sous tout autre gaz inerte vis-à-vis du milieu réactionnel.

La température réactionnelle de cette première étape est généralement comprise entre 0°C et 200°C suivant le solvant utilisé et de préférence entre 20°C et 120°C. Les durées et les pressions réactionnelles sont connues de l'homme de l'art et sont fonction du mode opératoire suivi.

Le produit issu du procédé selon l'invention peut réagir avec tous les composés susceptibles de réagir avec un alcoolate. Il peut être soumis à un traitement pour régénérer la fonction alcool ou pour modifier sa fonctionnalité.

On peut avantageusement profiter de sa basicité pour introduire une nouvelle fonctionnalité. Ainsi, par exemple, on pourra acidifier le milieu réactionnel pour fixer un proton ou rajouter un agent d'alkylation tel que le diméthylsulfate ou l'iodure de méthyle pour réaliser une alkylation. L'agent employé pour effectuer cette fonctionnalisation devra toutefois être choisi en tenant compte de la présence des fonctions amines, afin de ne pas les affecter.

Grâce au procédé selon l'invention, les rendements sont élevés et les produits finaux sont d'une pureté convenant à la plupart de leurs applications.

Le procédé selon l'invention permet donc de conserver les fonctions amines primaires ou secondaires de départ et ceci avantageusement en l'absence de toute protection préalable.

Elles sont donc susceptibles de donner lieu à toutes les réactions connues des amines primaires ou secondaires.

Les exemples suivants illustrent l'invention sans toutefois en limiter la portée.

### EXEMPLE 1:

**Synthèse du composé répondant à la formule :**

CH₃ - NH - (CH₂CH₂-O)₁₁-H

Dans un réacteur en verre parfaitement anhydre, on introduit sous argon 500 cm³ de tétrahydrofuranne anhydre et 8,05 cm³ de N-méthyléthanolamine (0,1 mole). Une solution de diphénylméthylpotassium, soit 106,15 cm³ d'une solution titrée exactement à 0,95 mole/l, est ajoutée goutte à goutte sous argon jusqu'à obtention d'une coloration persistante orange-brun du milieu. Il y a précipitation partielle de l'amino-alcoolate de potassium.

Les entrées et les sorties d'argon étant maintenues fermées, l'oxyde d'éthylène, soit 51 cm³ (1 mole à 20°C), est introduit sous forme liquide, puis le réacteur est porté à 30°C. Le réacteur est alors en surpression d'environ 0,2 bar et le mélange est maintenu agité à 30°C pendant 12 heures. Au terme de la réaction, le mélange prend une couleur cuivre. Il est acidifié par une solution de méthanol acide de 2 % en acide chlorhydrique.

La neutralisation est atteinte lorsque le milieu se décolore en jaune clair. On élimine le chlorure de potassium par filtration ; le tétrahydrofuranne (THF) est évaporé sous vide et le résidu est versé dans un mélange constitué de 800 cm³ d'eau et 800 cm³ d'éther.

La phase aqueuse contient le polymère aminé spécifiquement polyoxyéthylé sur l'oxygène de la fonction alcool, dont 51,5 g sont isolés après évaporation totale de l'eau sous vide.

Le spectre de RMN ¹H (résonance magnétique nucléaire du proton) dans le chloroforme deutérié est caractéristique du produit polyoxyéthylé à 11 unités d'oxyéthylène avec 1 singulet à 2,4 ppm (N-CH₃) (3H) ; 1 triplet à 2,7 ppm (N-CH₂) (2H) ; 1 singulet à 3,64 ppm (CH₂CH₂O)n (42H) et 1 singulet à 3,1 ppm (N-H et O-H) (2H).

L'absence de polymère produit par condensation de l'oxyde d'éthylène sur la fonction amine est démontrée par l'absence de singulet à 2,3 ppm qui correspondrait à la présence d'un méthyle sur un azote tertiaire. La chromatographie en perméation de gel (GPC) dans l'eau fournit un chromatogramme (préalablement étalonné par une gamme de polyoxyéthylène étalon) dont le maximum est situé à M_{w} = 520, en accord avec la masse attendue.

### EXEMPLE 2 :

**Synthèse du composé répondant à la formule :**

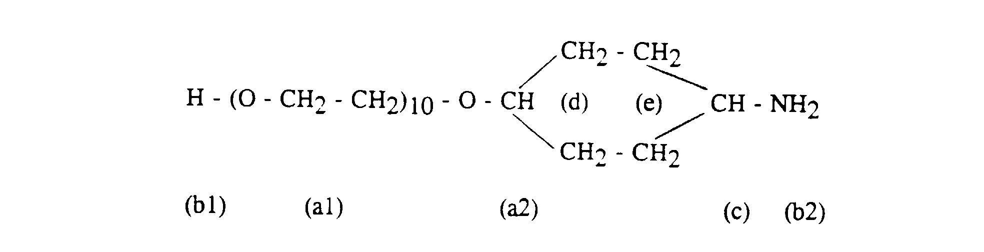

Selon le mode opératoire précédemment décrit dans l'exemple 1, 11,50 g (0,1 mole) d'amino-4 cyclohexanol sont dissous dans 500 ml de THF. Ensuite on ajoute 100 ml (0,1 mole) de diphénylméthylpotassium molaire dans le THF puis 51 ml (1 mole) d'oxyde d'éthylène. Après 20h de réaction à 30°C, le mélange est traité comme indiqué dans l'exemple 1.

Par RMN¹H on trouve les pics suivants :
- un multiplet à 3,64 ppm correspondant aux 41H désignés par a1 et a2
- un singulet à 3,10 ppm correspondant aux 3H désignés par b1 et b2
- un quintuplet à 2,7 ppm correspondant à 1H désigné par c
- un multiplet à 2,01 ppm correspondant aux 4H désignés par d
- un multiplet à 1,25 ppm correspondant aux 4H désignés par e.

Par GPC dans l'eau (chromatogramme étalonné comme précédemment), on trouve une masse moléculaire (Mw) égale à 550 g/mole.

### EXEMPLE 3 :

**Synthèse du composé répondant à la formule :**

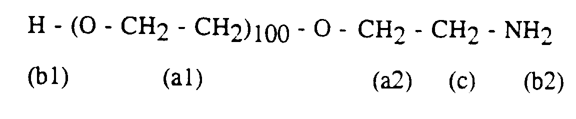

Ce composé est synthétisé selon le mode opératoire décrit dans l'exemple 1 à partir de 6,11 g d'éthanol-amine (0,01 mole), 10 ml de diphénylméthylpotassium molaire (0,01 mole) et 51 ml d'oxyde d'éthylène (1 mole).

Par RMN¹H on trouve les pics suivants :
- un multiplet à 3,64 ppm correspondant aux 402H désignés par a1 et a2
- un singulet à 3,05 ppm correspondant aux 3H désignés par b1 et b2
- un triplet à 2,70 ppm correspondant aux 2H désignés par c

Par GPC (H₂O), on trouve une masse moléculaire Mw égale à 4 200 g/mole.

### EXEMPLE 4 :

Selon le mode opératoire décrit dans l'exemple 1, on synthétise le produit répondant à la formule : 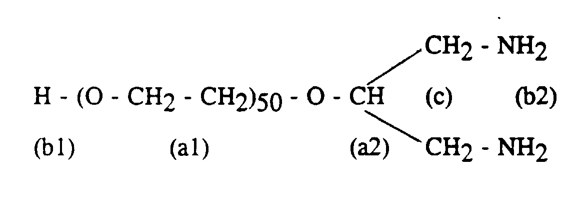 à partir de 1,8 g de diamino-propanol-2 (0,02 mole), 20 ml de diphénylméthylpotassium molaire (0,02 mole) et 51 ml d'oxyde d'éthylène (1 mole).

Par RMN¹H sur le produit obtenu, on trouve :
- un multiplet à 3,64 ppm correspondant aux 201H désignés par a1 et a2
- un singulet à 3,05 ppm correspondant aux 5H désignés par b1 et b2
- un multiplet à 2,70 ppm correspondant aux 4H désignés par c

Par GPC (H₂O), on trouve une masse moléculaire Mw égale à 2 250 g/mole.

### EXEMPLE 5 :

Selon le mode opératoire décrit dans l'exemple 1, on synthétise le produit répondant à la formule suivante : 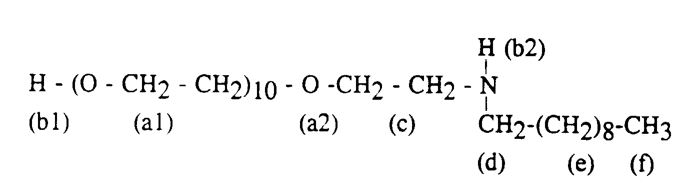 avec 10,05 g de N-décyléthanol amine (0,05 mole), 100 ml de diphénylméthylpotassium molaire (0,05 mole) et 51 ml d'oxyde d'éthylène (1 mole).

Par RMN¹H, on trouve le spectre suivant :
- un multiplet à 3,64 ppm correspondant aux 42H désignés par a1 et a2
- un singulet à 3,10 ppm correspondant aux 2H désignés par b1 et b2
- un triplet à 2,7 ppm correspondant aux 2H désignés par c
- un triplet à 2,4 ppm correspondant aux 2H désignés par d
- un multiplet à 1,35 ppm correspondant aux 16H désignés par e
- un triplet à 0,95 ppm correspondant aux 3H désignés par f.

Par GPC (H₂O), on trouve une masse moléculaire Mw égale à 1 050 g/mole.

### EXEMPLE 6 :

**Polyoxyalkylation à partir d'un composé comportant des fonctions alcoolates de sodium :**

On procède comme indiqué dans l'exemple 1 avec 7,51 g de N-méthyléthanol amine (0,1 mole), 100 ml de diphénylméthylsodium molaire (0,1 mole) et 51 ml d'oxyde d'éthylène (1 mole).

Après 20h de réaction, la conversion d'oxyde d'éthylène est de 55 %.

Par RMN¹H, on trouve le spectre suivant :
- 1 singulet à 2,43 ppm correspondant à un méthyle fixé sur un azote secondaire
- 1 singulet à 2,31 ppm correspondant à un méthyle fixé sur un azote tertiaire.

Le taux d'amine tertiaire est de 40 % (en mole).

### EXEMPLE 7 :

**Polyoxyalkylation à partir d'un mélange obtenu par conversion de 10 % des groupements OH en groupements OK :**

On opère selon le mode opératoire décrit dans l'exemple 1, avec 6,11 g d'éthanol amine (0,1 mole), 10 ml de diphénylméthylpotassium molaire (0,01 mole) et 51 ml d'oxyde d'éthylène (1 mole).

Après 120 h de réaction à 30°C, la conversion d'oxyde d'éthylène n'est que de 22 % , le taux d'amine secondaire est de 28 % (en mole) et le taux d'amine tertiaire est de 19 % (en mole).

### EXEMPLE 8 :

**Polyoxyalkylation à partir d'un mélange obtenu par conversion de 50 % des groupements OH en groupements OK :**

On opère à nouveau selon le mode opératoire décrit dans l'exemple 1 avec 6,11 g d'éthanol amine (0,1 mole), 50 ml de diphénylméthylpotassium molaire (0,05 mole) et 51 ml d'oxyde d'éthylène (1 mole).

Après 20 h de réaction à 30°C, la conversion d'oxyde d'éthylène est de 95 %. Le taux d'amine primaire est de 93 %, celui d'amine secondaire de 7 % (exprimé en mole).

### EXEMPLE 9 :

**Polyoxyalkylation à partir d'un mélange obtenu par conversion de 75 % des groupements OH en groupements OK :**

Selon le mode opératoire décrit dans l'exemple 1, on synthétise le produit correspondant à la formule suivante : 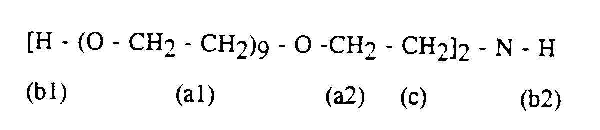 50 ml de diphénylméthylpotassium molaire (0,05 mole) sont introduits dans 500 cm³ de THF, suivis de 5,25 g de diéthanolamine (0,05 mole) puis de 25 ml de solution de diphénylméthylpotassium (0,025 mole). Enfin, 51 ml d'oxyde d'éthylène sont ajoutés.

On procède comme dans l'exemple à l'exception près que l'on laisse réagir pendant 20 heures :
Le spectre RMN¹H du produit obtenu est le suivant :
- un multiplet à 3,64 ppm correspondant aux 76H désignés par a1 et a2
- un singulet à 3,15 ppm correspondant aux 3H désignés par b1 et b2
- un triplet à 2,65 ppm correspondant aux 4H désignés par c

Le produit contient alors environ 5 % en mole d'amine tertiaire.

La masse moléculaire obtenue par GPC est égale à 900 g/mole.

## Revendications

1. Procédé de préparation de composés comportant au moins une fonction amine primaire ou secondaire et au moins une chaîne polyoxyalkylée, caractérisé en ce que l'on fait réagir :
- au moins un amino-alcoolate (B) obtenu à partir d'un amine-alcool (A) comportant au moins un groupement OH et au moins une fonction amine primaire ou secondaire, par remplacement d'une fraction ou de la totalité des groupements OH par des groupements OM, M étant un atome choisi parmi le sodium, le potassium, le rubidium et le césium, lesdits amino-alcool (A) et amino-alcoolate (B) étant dépourvus de groupements susceptibles de réagir entre eux ou avec une fonction portée par l'amino-alcool (A) ou l'amino-alcoolate (B) dans les conditions réactionnelles du procédé ;
- avec un oxirane, un mélange d'oxiranes ou une séquence d'oxiranes, dans un solvant aprotique et anhydre, sous atmosphère anhydre et à température comprise entre 0°C et 200°C, de façon à fixer sur chaque atome d'oxygène provenant, soit d'un groupement OH, soit d'un groupement OM, une série de "m" motifs semblables ou différents, m étant un nombre entier supérieur ou égal à 1, de préférence compris entre 1 et 10 000, lesdits "m" motifs répondant à la formule :
-[C (R₁)(R₂) -C (R₃)(R₄) - O]-
- dans laquelle R₁, R₂, R₃ et R₄ sont semblables ou différents et peuvent être choisis dans le groupe constitué par :
* l'atome d'hydrogène
* les radicaux monovalents, saturés ou non, alkyle, cyclo-alkyle ou aryle,
* lesdits radicaux renfermant éventuellement divers substituants non réactifs vis-à-vis d'une fonction amine et d'une fonction alcoolate.

2. Procédé selon la revendication 1, caractérisé en ce que le nombre "m" de motifs est compris entre 1 et 500, spécialement entre 1 et 250.

3. Procédé selon la revendication 1 ou la revendication 2, caractérisé en ce que M est choisi parmi le potassium, le rubidium et le césium.

4. Procédé selon la revendication 3, caractérisé en ce que M est le potassium.

5. Procédé selon l'une quelconque des revendications 1 à 4, caractérisé en ce que l'amino-alcool (A) répond à la formule (III) suivante :
[(HNRi)ⱼQ](OH)_{(r+q)}
dans laquelle :
- j est le nombre de fonctions amines primaires ou secondaires qui sont identiques ou différentes les unes des autres, j variant de 1 à 10 inclus,
- r est la somme des nombres de groupements OH portés par l'ensemble des Ri et q est le nombre des groupemens OH portés par Q, r+q étant supérieur ou égal à 1 et pouvant atteindre 10,
- Q représente un radical organique comportant de 2 à 50 atomes de carbone,
- chaque Ri est un atome d'hydrogène ou un radical organique comportant de 1 à 50 atomes de carbone,
- quand l'amino-alcool (A) renferme plus d'une fonction amine primaire ou secondaire, les Ri peuvent être semblables ou différents entre eux,
- Q et Ri peuvent être porteurs d'hétéroatomes et/ou comporter des substituants divers,
- Q, N et les Ri peuvent former ensemble un ou plusieurs cycles, ce ou ces cycles pouvant en outre contenir un ou plusieurs autres hétéroatomes,
- Q et les Ri doivent être dépourvus de groupements susceptibles de réagir entre eux ou avec d'autres groupements portés par l'amino-alcool (A) ou l'amino-alcoolate (B) dans les conditions réactionnelles du procédé selon l'invention.

6. Procédé selon l'une quelconque des revendications 1 à 5, caractérisé en ce que le nombre des groupements OM est supérieur à 30 %, de préférence supérieur à 40 % de la somme du nombre des groupements OM et du nombre des groupements OH.

7. Procédé selon l'une quelconque des revendications 1 à 6, caractérisé en ce que l'oxirane utilisé dans la première étape est choisi dans le groupe constitué par l'oxyde d'éthylène, l'oxyde de propylène, l'oxyde de butylène, l'oxyde d'amylène, l'oxyde d'octylène, l'oxyde de styrène, l'oxyde de méthylstyrène, l'oxyde de cyclohexane, leurs divers dérivés substitués et leurs mélanges.

8. Procédé selon la revendication 7, caractérisé en ce que l'oxirane est choisi dans le groupe constitué par l'oxyde d'éthylène, l'oxyde de propylène, l'oxyde de styrène et leurs mélanges.

9. Procédé selon la revendication 8, caractérisé en ce que l'oxirane est l'oxyde d'éthylène.

10. Procédé selon l'une quelconque des revendications 1 à 9, caractérisé en ce que le solvant aprotique et anhydre est choisi parmi le tétrahydrofuranne, le diglyme, le diméthylsulfoxyde ou l'hexaméthylphosphoreamide.

11. Procédé selon l'une quelconque des revendications 1 à 10, caractérisé en ce que l'addition d'oxirane est réalisée à température comprise entre 20°C et 120°C.

12. Procédé selon l'une quelconque des revendications 1 à 11, caractérisé en ce que l'amino-alcool (A) est choisi parmi les composés suivants :
- l'éthanol-amine
- la diéthanol-amine
- les propanol-amines
- la N-méthyléthanolamine
- l'amino-4-cyclohexanol
- le diamino-propanol-2
- la N-décyléthanolamine.

13. Procédé selon l'une quelconque des revendications 1 à 12, caractérisé en ce qu'il est suivi d'une étape de neutralisation ou de fonctionnalisation de chaque atome d'oxygène terminant une chaîne polyoxyalkylée.

14. Procédé selon l'une quelconque des revendications 1 à 13, caractérisé en ce qu'il est suivi d'une étape au cours de laquelle on fait réagir, de façon connue, au moins une des fonctions amines primaires ou secondaires du produit obtenu.

## Patentansprüche

1. Verfahren zur Herstellung von Verbindungen, die mindestens eine primäre oder sekundäre Aminfunktion und mindestens eine polyoxyalkylierte Kette aufweisen, dadurch gekennzeichnet, daß man
- mindestens ein Aminoalkoholat (B), hergestellt aus einem Aminoalkohol (A), der mindestens eine OH-Gruppe und mindestens eine primäre oder sekundäre Aminfunktion aufweist, durch Ersatz eines Teils oder der Gesamtmenge der OH-Gruppen durch OM-Gruppen, worin M für ein Atom, ausgewählt aus der Gruppe Natrium, Kalium, Rubidium und Cäsium, steht, wobei der genannte Aminoalkohol (A) und das genannte Aminoalkoholat (B) frei von Gruppen sind, die untereinander oder mit einer Funktion, die der Aminoalkohol (A) oder das Aminoalkoholat (B) aufweist, unter den Reaktionsbedingungen des Verfahrens reagieren können;
- mit einem Oxiran, einer Mischung von Oxiranen oder einer Sequenz von Oxiranen in einem aprotischen und wasserfreien Lösungsmittel unter einer wasserfreien Atmosphäre und bei einer Temperatur zwischen 0 und 200°C reagieren läßt, um so an jedes Sauerstoffatom, das entweder aus einer OH-Gruppe oder aus einer OM-Gruppe stammt, eine Reihe von "m" gleichen oder unterschiedlichen wiederkehrenden Einheiten zu binden, wobei m eine ganze Zahl ≥ 1 ist, die vorzugsweise zwischen 1 und 10 000 liegt, und die genannten "m" wiederkehrenden Einheiten der Formel entsprechen:
-[C(R₁)(R₂) - C(R₃)(R₄) - O]-
worin R₁, R₂, R₃ und R₄ gleich oder verschieden sind und ausgewählt werden aus der Gruppe, die besteht aus:
* einem Wasserstoffatom,
* monovalenten gesättigten oder ungesättigten Alkyl-, Cycloalkyl- oder Aryl-Resten,
* wobei die genannten Reste gegebenenfalls verschiedene Substituenten tragen, die mit einer Aminfunktion und einer Alkoholatfunktion nicht reagieren.

2. Verfahren nach Anspruch 1, dadurch gekennzeichnet, daß die Anzahl "m" der wiederkehrenden Einheiten zwischen 1 und 500, insbesondere zwischen 1 und 250, liegt.

3. Verfahren nach Anspruch 1 oder 2, dadurch gekennzeichnet, daß M ausgewählt wird aus der Gruppe Kalium, Rudibium und Cäsium.

4. Verfahren nach Anspruch 3, dadurch gekennzeichnet, daß M für Kalium steht.

5. Verfahren nach einem der Ansprüche 1 bis 4, dadurch gekennzeichnet, daß der Aminoalkohol (A) der folgenden Formel (III) entspricht:
[HNRi)ⱼQ](OH)_{(r+q)}
worin bedeuten:
j die Anzahl der primären oder sekundären Aminfunktionen, die identisch oder voneinander verschieden sind, wobei j zwischen 1 und einschließlich 10 variiert,
r die Summe der Anzahl der OH-Gruppen, die von der Gesamtheit der Reste Ri getragen werden, und
q die Anzahl der OH-Gruppen, die von Q getragen werden, wobei r + q ≥ 1 ist und bis zu 10 erreichen kann,
Q einen organischen Rest, der 2 bis 50 Kohlenstoffatome aufweist,
jeder Rest Ri ein Wasserstoffatom oder einen organischen Rest, der 1 bis 50
Kohlenstoffatome aufweist, wobei
dann, wenn der Aminoalkohol (A) mehr als eine primäre oder sekundäre Aminfunktion aufweist, die Reste Ri gleich oder voneinander verschieden sein können,
Q und Ri Heteroatome und/oder verschiedene Substituenten tragen können, Q, N und die Reste Ri gemeinsam einen oder mehrere Ringe bilden können, wobei dieser Ring oder diese Ringe außerdem ein oder mehrere andere Heteroatome aufweisen kann (können), und
Q und die Reste Ri frei von Gruppen sein müssen, die untereinander oder mit anderen Gruppen, die der Aminoalkohol (A) oder das Aminoalkoholat (B) aufweist, unter den Reaktionsbedingungen des erfindungsgemäßen Verfahrens reagieren können.

6. Verfahren nach einem der Ansprüche 1 bis 5, dadurch gekennzeichnet, daß die Anzahl der OM-Gruppen mehr als 30 %, vorzugsweise mehr als 40 %, der Summe aus der Anzahl der OM-Gruppen und der Anzahl der OH-Gruppen beträgt.

7. Verfahren nach einem der Ansprüche 1 bis 6, dadurch gekennzeichnet, daß das in der ersten Stufe verwendete Oxiran ausgewählt wird aus der Gruppe, die besteht aus Ethylenoxid, Propylenoxid, Butylenoxid, Amylenoxid, Octylenoxid, Styroloxid, Methylstyroloxid, Cyclohexanoxid, ihren verschiedenen substituierten Derivaten und ihren Mischungen.

8. Verfahren nach Anspruch 7, dadurch gekennzeichnet, daß das Oxiran ausgewählt wird aus der Gruppe, die besteht aus Ethylenoxid, Propylenoxid, Styroloxid und ihren Mischungen.

9. Verfahren nach Anspruch 8, dadurch gekennzeichnet, daß das Oxiran das Ethylenoxid ist.

10. Verfahren nach einem der Ansprüche 1 bis 9, dadurch gekennzeichnet, daß das aprotische und wasserfreie Lösungsmittel ausgewählt wird aus der Gruppe Tetrahydrofuran, Diglyme, Dimethylsulfoxid und Hexamethylphosphoramid.

11. Verfahren nach einem der Ansprüche 1 bis 10, dadurch gekennzeichnet, daß die Zugabe von Oxiran bei einer Temperatur zwischen 20 und 120°C erfolgt.

12. Verfahren nach einem der Ansprüche 1 bis 11, dadurch gekennzeichnet, daß der Aminoalkohol (A) unter den folgenden Verbindungen ausgewählt wird:
- Ethanolamin
- Diethanolamin
- den Propanolaminen
- N-Methylethanolamin
- Amino-4-cyclohexanol
- Diamino-propanol-2
- N-Decylethanolamin.

13. Verfahren nach einem der Ansprüche 1 bis 12, dadurch gekennzeichnet, daß sich daran eine Stufe zur Neutralisation oder Funktionalisierung jedes endständigen Sauerstoffatoms einer polyoxyalkylierten Kette anschließt.

14. Verfahren nach einem der Ansprüche 1 bis 13, dadurch gekennzeichnet, daß sich eine Stufe anschließt, in deren Verlauf man auf an sich bekannte Weise mindestens eine der primären oder sekundären Aminfunktionen des erhaltenen Produkts reagieren läßt.

## Claims

1. Process for the preparation of compounds having at least one primary or secondary amine function and at least one polyoxyalkylated chain, characterised in that
- at least one amino alcoholate (B), obtained from an amino alcohol (A) comprising at least one OH group and having at least one primary or secondary amine function, by replacing a fraction of or all the OH groups with OM groups, M being an atom selected from sodium, potassium, rubidium and caesium, said amino alcohols (A) and the amino alcoholate (B) being devoid of groups capable of reacting with one another, or with a function carried by the amino alcohol (A) or the amino alcoholate (B) in the reactive conditions of the process;
is caused to react with
- an oxiran, a mixture of oxirans or an oxirans sequence, in an aprotic and anhydrous solvent, in an anhydrous atmosphere and at a temperature of between 0°C and 200°C, in order to attach to each atom of oxygen originating either from an OH group or an OM group a series of "m" motifs, either the same or different, m being a whole number greater than or equal to 1, preferably lying between 1 and 10 000, said "m" motifs corresponding to the following formula:
-[C(R₁)(R₂) - C(R₃)(R₄) - O]-
wherein R₁, R₂, R₃ and R₄ are the same or different, and may be selected from within the group comprising:
* the hydrogen atom
* monovalent radicals, either saturated or not, alkyl, cyclo-alkyl or aryl,
* said radicals possibly including various substituents which are non-reactive with respect to an amine function or an alcoholate function.

2. Process according to Claim 1, characterised in that the number "m" of motifs lies between 1 and 500, in particular between 1 and 250.

3. Process according to Claim 1 or Claim 2, characterised in that M is selected from potassium, rubidium and caesium.

4. Process according to Claim 3, characterised in that M is potassium.

5. Process according to any one of Claims 1 to 4, characterised in that the amino alcohol (A) corresponds to the following formula (III):
[(HNRi)ⱼQ](OH)_{(r+q)}
wherein:
- j is the number of primary or secondary amine functions, which are identical to or different from one another, j varying from 1 to 10 inclusive,
- r is the sum of the numbers of OH groups carried by all the Ri components, and q is the number of OH groups carried by Q, r+q being greater than or equal to 1 and may be as much as 10,
- Q represents an organic radical containing 2 to 50 carbon atoms,
- each Ri is a hydrogen atom or an organic radical containing 1 to 50 carbon atoms,
- when the amino alcohol (A) includes more than one primary or secondary amine function, the Ri components may be the same or different from one another,
- Q and Ri can be carriers of hetero-atoms and/or have various substituents,
- Q, N and the Ri components may together form one or several cycles, and this cycle or these cycles may, moreover, contain one or several other hetero-atoms,
- Q and the Ri components must be devoid of groups capable of reacting with one another or with other groups carried by the amino alcohol (A) or the amino alcoholate (B) in the reactive conditions of the process according to the invention.

6. Process according to any one of Claims 1 to 5,
characterised in that the number of OM groups is greater than 30%, preferably greater than 40%, of the sum of the number of OM groups and the number of OH groups.

7. Process according to any one of Claims 1 to 6,
characterised in that the oxiran used in the first stage is selected from the group comprising ethylene oxide, propylene oxide, butylene oxide, amylene oxide, octylene oxide, styrene oxide, methyl styrene oxide, cyclohexane oxide, their various substituted derivatives, and mixtures thereof.

8. Process according to Claim 7, characterised in that the oxiran is selected from the group comprising ethylene oxide, propylene oxide, styrene oxide and mixtures thereof.

9. Process according to Claim 8, characterised in that the oxiran is ethylene oxide.

10. Process according to any one of Claims 1 to 9,
characterised in that the aprotic and anhydrous solvent is selected from tetrahydrofuran, diethyl glycol dimethyl ether, dimethylsulphoxide or hexamethylphosphoreamide.

11. Process according to any one of Claims 1 to 10,
characterised in that the addition of oxiran is carried out at a temperature between 20°C and 120°C.

12. Process according to any one of Claims 1 to 11,
characterised in that the amino alcohol (A) is selected from the following components:
- ethanolamine
- diethanolamine
- propanol amines
- N-methyl ethanolamine
- amino-4-cyclohexanol
- diamino-propanol-2
- N-decyl ethanolamine.

13. Process according to any one of Claims 1 to 12,
characterised in that it is followed by a stage of rendering each oxygen atom terminating a polyoxyalkylated chain neutral or functional.

14. Process according to any one of Claims 1 to 13,
characterised in that it is followed by a stage, during which at least one of the primary or secondary amine functions of the product obtained is caused to react in a known manner.
